# EUROPEAN PATENT SPECIFICATION

(11) **EP 1 656 908 B1**
(45) Date of publication and mention of the grant of the patent: **19.12.2007**
(21) Application number: 05024576.0
(22) Date of filing: 10.11.2005
(51) Int. Cl.: A61F 2/06

(54) **Stent crimper with slit sheath**
Stentquetschwerkzeug mit geschlitzter Hülle
Sertisseur de stents avec une gaine à fentes

(30) Priority: 10.11.2004 US 985560
(43) Date of publication of application: 17.05.2006
(73) Proprietor: Medtronic Vascular, Inc., Santa Rosa, CA 95403 (US)
(72) Inventor: Pryor, Jack, San Diego, CA 92129 (US)
(74) Representative: Zimmermann, Gerd Heinrich

(56) References cited:
- WO-A-20/04019768
- WO-A-20/05070336
- US-A1- 2001 001 890
- US-A1- 2004 181 236
- US-B1- 6 651 487

## Description

### TECHNICAL FIELD

This invention relates generally to biomedical systems for treating vascular conditions and to systems and methods for manufacturing such biomedical systems. More specifically, the invention relates to a method and system for crimping a self-expandable stent while at the same time partially or wholly enclosing the stent in a slit sheath, and to a system for treating a vascular condition produced using the crimping and sheathing system and method.

### BACKGROUND OF THE INVENTION

Stents are cylindrical-shaped devices that are radially expandable to hold open a segment of a vessel or other anatomical lumen after implantation into the lumen. Various types of stents are in use, including expandable and self-expanding stents. Expandable stents generally are conveyed to the area to be treated on balloon catheters or other expandable devices. For a self-expanding stent, commonly a sheath is retracted, allowing expansion of the stent.

Stent insertion may cause undesirable reactions such as inflammation, infection, thrombosis, and proliferation of cell growth that occludes the passageway. Stents have been used with coatings to deliver drugs or other therapeutic agents at the site of the stent that may assist in preventing these conditions. The coatings must be bioengineered to control the release of highly potent and potentially toxic drugs.

Often a cap coating is deposited over one or more drug coatings to provide a carefully timed release of the drug(s). Protecting the cap coating from abrasion or other damage is important to ensure the desired delivery of the drug(s). Retracting a sheath from a self-expanding stent may scratch a coating deposited on the stent. Therefore, it would be desirable to have an improved system for treating a vascular condition and a manufacturing system and method for producing such a treatment system that overcomes the aforementioned and other disadvantages.

### SUMMARY OF THE INVENTION

One aspect of the present invention is a system for crimping and sheathing a stent. The system comprises a radial compression device including a plurality of crimping members. At least a plurality of the crimping members include a contoured depression. An inner sheath segment is received in each contoured depression. The depression holds the sheath segment while the compression device moves between an open and a closed position.

Another aspect of the present invention is a system for treating a vascular condition. The system comprises a crimped stent at least partially enclosed within a segmented sheath member. The stent was simultaneously crimped and sheathed by segments of the sheath member held within contoured depressions formed in crimping members of a radial compression device.

Yet another aspect of the present invention is a method of crimping and sheathing a stent. A plurality of inner sheath segments are positioned in contoured depressions formed in crimping members of a radial compression device. A stent is positioned within the radial compression device while the device is in an open position. The radial compression device is closed to crimp the stent and to at least partially enclose the stent in the inner sheath segments. The crimped stent and inner sheath segments are slid into an outer sheath.

The aforementioned and other features and advantages of the invention will become further apparent from the following detailed description of the presently preferred embodiments, read in conjunction with the accompanying drawings. The detailed description and drawings are merely illustrative of the invention rather than limiting, the scope of the invention being defined by the appended claims and equivalents thereof.

A system for crimping a stent according to the preamble of claim 1 is known from the documents US-A-2004/181236 and US-A-6651487,

### BRIEF DESCRIPTION OF THE DRAWINGS

**FIGS. 1A** and **1B** are illustrations of one embodiment of a system for crimping and sheathing a stent, in accordance with the present invention;

**FIGS. 2A** and **2B** are isometric views of the system of **FIGS. 1A** and **1B;**

**FIGS. 3A** and **3B** are illustrations of another embodiment of a system for crimping and sheathing a stent, in accordance with the present invention, the system being capable of crimping a stent from a large expanded diameter to a small crimped diameter;

**FIGS. 4A** and **4B** are illustrations of another embodiment of a system for crimping and sheathing a stent, in accordance with the present invention, each sheath segment being held in a shape lock depression within a crimping member;

**FIGS. 5A** and **5B** are illustrations of another embodiment of a system for crimping and sheathing a stent, in accordance with the present invention, each sheath segment being held by an alternative shape lock depression within a crimping member;

**FIGS. 6A** and **6B** are illustrations of another embodiment of a system for crimping and sheathing a stent, in accordance with the present invention, the crimping members shown as substantially triangular in cross section;

**FIGS. 7A** and **7B** are illustrations of another embodiment of a system for crimping and sheathing a stent, in accordance with the present invention, each sheath segment having a spline to aid in holding the segment in a complementary contoured depression within a crimping member;

**FIGS. 8A** and **8B** are illustrations of an embodiment of a system for treating a vascular condition, in accordance with the present invention; and

**FIG. 9** is a flow diagram of one embodiment of a method of crimping and sheathing a stent, in accordance with the present invention.

### DETAILED DESCRIPTION OF THE PRESENTLY PREFERRED EMBODIMENTS

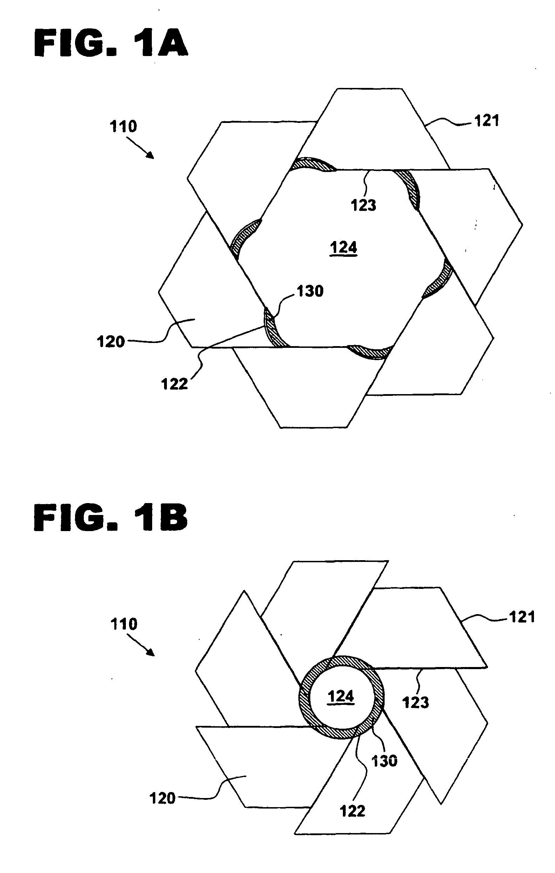

One aspect of the present invention is a system for crimping and sheathing a stent. One embodiment of the system, in accordance with the present invention, is illustrated in **FIGS. 1A-2B.** The system comprises a radial compression device **110** that includes multiple crimping members **120.** Each crimping member includes a contoured depression **122.** Inner sheath segments **130** are received in the contoured depressions.

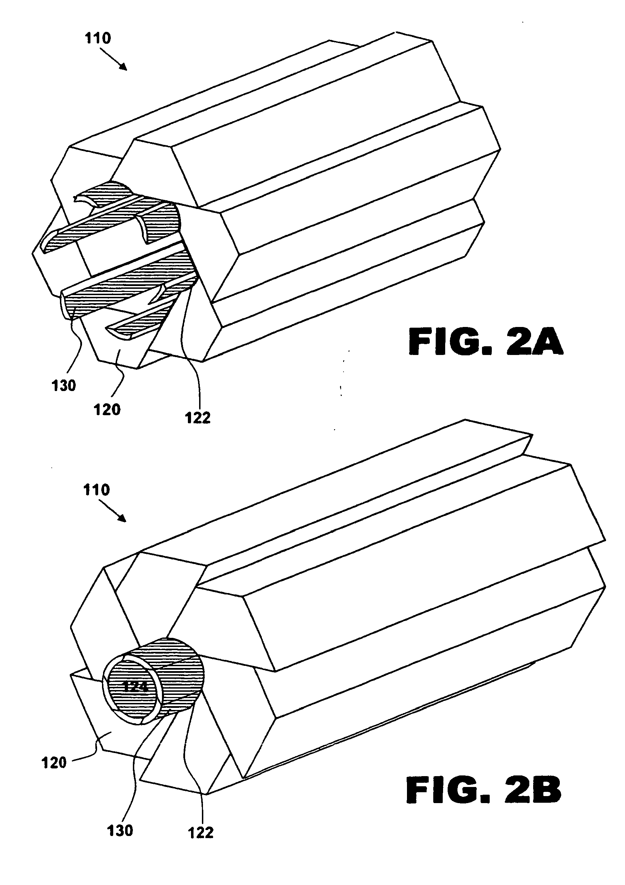

As illustrated, radial compression device 110 includes six crimping members **120;** however, the number of crimping members is variable. As is best seen in **FIGS. 2A** and **2B,** each crimping member **120** is an elongated structure, with the length of the crimping member selected such that the member extends over the entire length of the stent to be crimped. In cross section, or as seen end-on as in **FIGS. 1A** and **1B,** the crimping members are roughly trapezoidal, with a contoured depression **122** formed into each crimping member to hold an inner sheath segment **130.** In another embodiment, not all of the crimping members may hold an inner sheath segment, with the remaining crimping members being designed to duplicate the shape formed by a crimping member plus a sheath segment.

Crimping members **120** may be formed from any appropriate material known in the art that is hard enough to crimp a stent and also formable to include a contoured depression. Such materials include, but are not limited to, stainless steel and a hard polymer. The material should be capable of being smoothed or polished to provide surfaces within contoured depressions **122** that allow inner sheath segments **130** to be easily removed from radial compression device **110** by sliding the segments out of the contoured depressions when the device is in a closed position.

Radial compression device **110** functions like a conventional variable diameter diaphragm, also called an iris diaphragm. In the present embodiment, crimping members **120** are arranged about the circumference of a circle with a side surface **121** of each crimping member in contact with a bottom surface **123** of an adjoining crimping member. The crimping members form a central aperture **124** having a variable diameter. Contoured depressions **122** of crimping members **120** face into this central aperture.

Each crimping member 120 rotates about a different longitudinal axis as compression device **110** moves between an open and a closed position. Rotation of the crimping members causes contoured depressions **122** to extend into central aperture **124** when the device moves from an open position to a closed position, thereby reducing the diameter of aperture **124** as the device closes.

Crimping members **120** form a central aperture sized to receive an expanded stent when compression device **110** is in an open position as seen in **FIGS. 1A** and **2A.** The crimping members form a substantially cylindrical central aperture sized to crimp the stent when the compression device is in a closed position as seen in **FIGS. 1B and 2B.** As illustrated in **FIG. 2B,** sheath segments **130** held within contoured depressions **122** are positioned to fully enclose a crimped stent when compression device **110** is in a closed position. Sheath segments **130** are shown in **FIGS. 2A** and **2B** as extending beyond crimping members **120** but may instead be fully contained within the compression device during operation of the device.

Sheath segments **130** may be manufactured from an appropriate biocompatible material, including, but not limited to, stainless steel, nitinol, polypropylene, polyethylene, a nylon/polyethylene blend, polytetrafluoroethylene (PTFE), a suitable polycarbonate, and combinations of the above. The segments may be formed by cutting evenly spaced longitudinal slits into a section of tubing, or each segment may be formed individually by, for example, extruding, molding, cutting, or stamping the segments. When cut from a section of tubing, the segments will be arc shaped in cross section. When formed by a method such as extruding or cutting, the segments may assume a variety of shapes in cross section, and may even be flat strips that when assembled form a substantially cylindrical structure.

Sheath segments 130 may be held within contoured depressions **122** by a variety of methods. For example, the sheath segments may be held by suction that is discontinued once compression device **110** has completed crimping and sheathing a stent, allowing the sheath segments and crimped stent to be easily removed from the compression device by sliding the sheathed stent out of the closed device. Alternatively, the sheath segment surface in contact with a contoured depression may be coated with a biocompatible material that lightly adheres to a crimping member while the compression device moves between an open position and a closed position and also serves as a lubricant to aid in sliding the sheath segment into and out of the crimping member. As another example, the sheath segments may be held by weak electrostatic attraction between the sheath segments and the crimping members that is produced by methods known in the art.

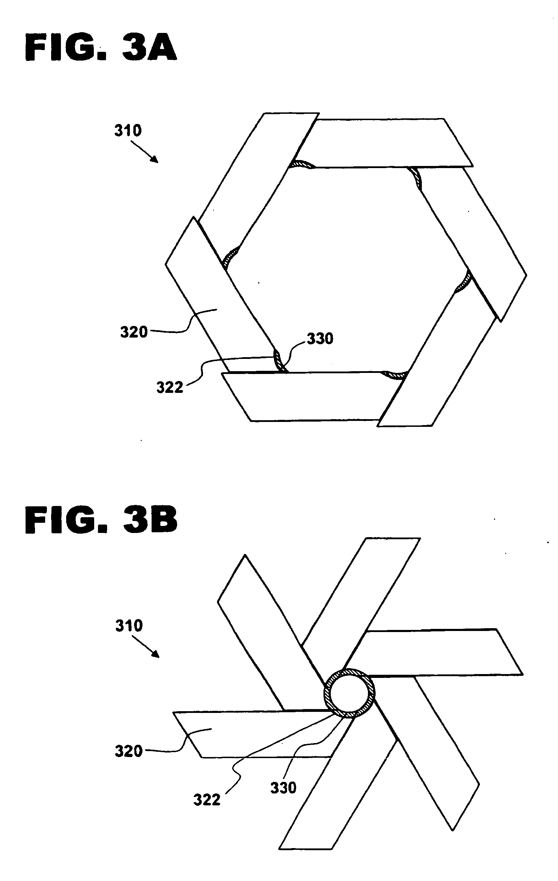

**FIGS. 3A-7B** show alternative embodiments in accordance with the present invention. The system shown open in **FIG. 3A** and closed in **FIG. 3B** is similar to that shown in **FIGS. 1A-2B,** having similarly shaped contoured depressions **322** and sheath segments **330,** but with the dimensions of the depressions, segments, and crimping members **320** sized to permit a stent to be crimped from a larger expanded diameter to a smaller crimped diameter using compression device **310** than would be possible with compression device **110.**

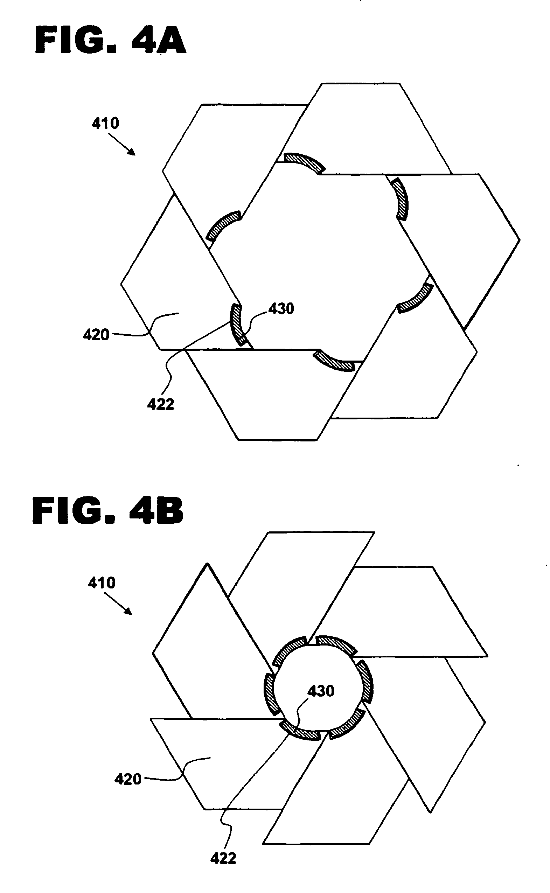

**FIGS. 4A** and **4B** show a system in which contoured depressions **422** of crimping members **420** serve as shape locks for sheath segments **430.** The sheath segments are held by the shape locks while compression device **410** moves between an open and a closed position but may be slidably removed from the device when the device is closed. In this example, sheath segments **430** are positioned to partially enclose a crimped stent when compression device **410** is in a closed position.

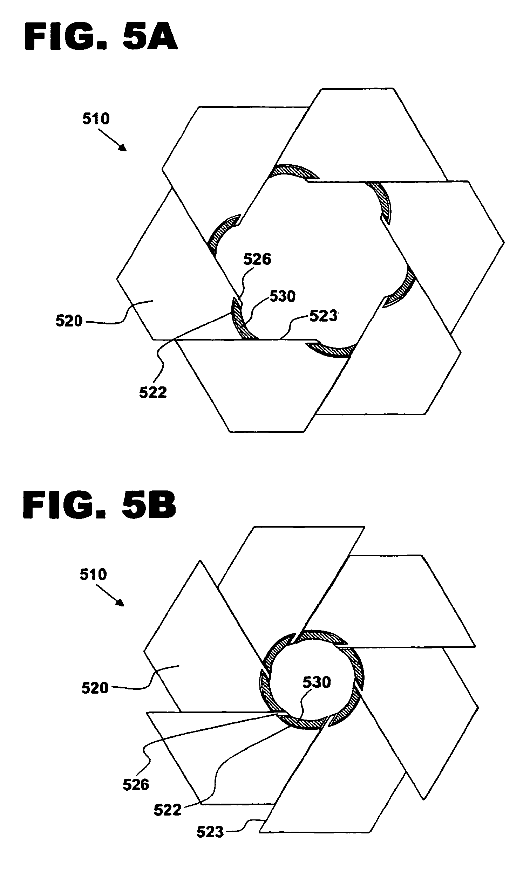

**FIGS. 5A** and **5B** show another version of a shape lock. In this example, each sheath segment **530** is held within a contoured depression **522** by a combination of a tab **526** holding one side of the segment and the bottom surface **523** of the adjoining crimping member **520** abutting the opposite side of the segment, thereby holding the segments while compression device **510** moves from an open to a closed position. As in the system described above and illustrated in **FIGS. 4A** and **4B,** sheath segments **530** are positioned to partially enclose a crimped stent when compression device **510** is in a closed position.

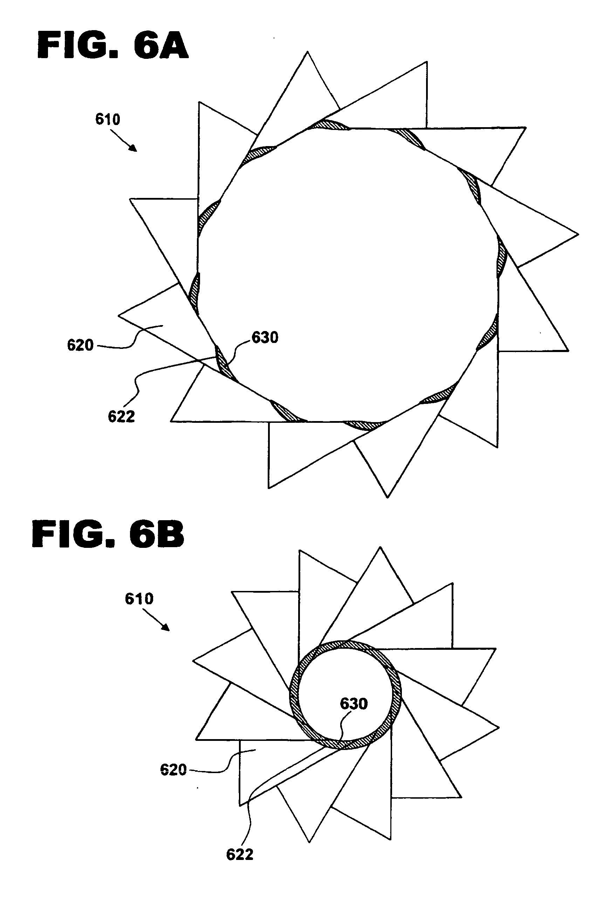

**FIGS. 6A** and **6B** show a system with a compression device **610** having twelve crimping members **620** that are substantially triangular in cross section. As is illustrated by these figures, a system in accordance with the present invention may have various numbers of crimping members, and the crimping members need not be trapezoidal but may be any shape that permits sheath segments held within contoured depressions in the crimping members to be rotated into a central aperture formed by the crimping members, thereby reducing the radius of the aperture and simultaneously crimping and sheathing a stent. As illustrated in **FIG. 6B,** sheath segments **630** held within contoured depressions **622** are positioned to fully enclose a crimped stent when compression device **610** is in a closed position.

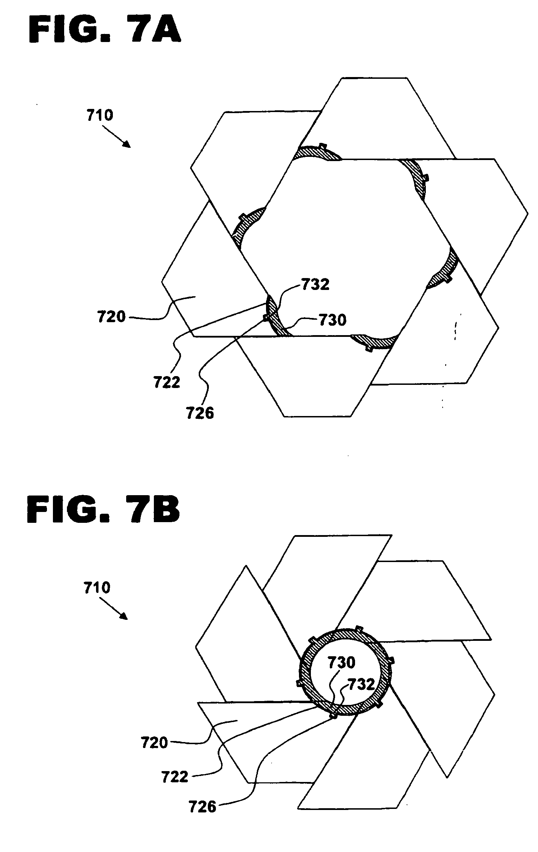

**FIG. 7** shows a system having sheath segments **730** that include splines **732.** The splines fit into complementary recesses **726** formed in contoured depressions **722.** The splines aid in holding the segments as crimping members **720** rotate to transition compression device **710** between an open and a closed position.

In practice, the above-described systems are capable of crimping a stent in a substantially uniform manner along the full length of the stent. The invention allows stents of any diameter and length to be simultaneously crimped and at least partially enclosed within a segmented sheath. The sheath segments protect the crimped stent from abrasion as the stent is slid out of the compression device and offer the added advantage of allowing a crimped stent to be removed from the compression device by pulling on a proximal end of the segmented sheath rather than by pushing the sheathed stent out of the device. For example, a stent that does not have sufficient column strength or rigidity to be pushed out of the compression device may instead be pulled from the device, eliminating the risk of longitudinal compression or buckling of the stent. A combination of pushing on the stent and pulling on the segmented sheath may be employed if desired.

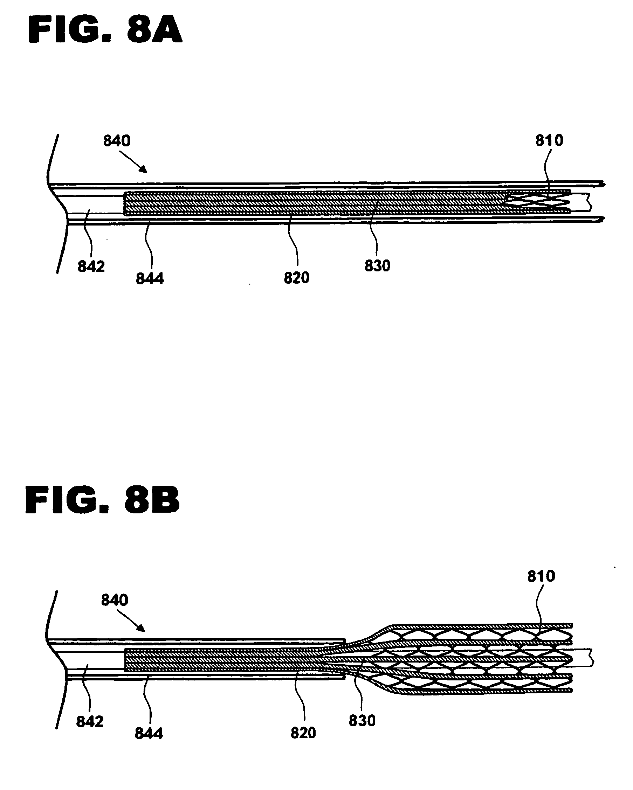

Another aspect of the present invention is a system for treating a vascular condition. One embodiment of the system, in accordance with the present invention, is illustrated in **FIGS. 8A** and **8B.** The system comprises a crimped stent **810,** a segmented sheath member **820** comprising multiple sheath segments **830,** and a catheter **840** having an inner member **842** and an outer member **844.**

In the present embodiment, crimped stent **810** is a coated, self-expanding, nitinol stent approximately 10 millimeters in expanded diameter and approximately 80 millimeters in length. However, the stent may be any size or design of self-expanding stent known in the art, manufactured from, for example, another nickel-titanium alloy, a nickel-cobalt alloy, another cobalt alloy, a thermoset plastic, stainless steel, a stainless steel alloy, or another biocompatible material.

Crimped stent **810** is shown in **FIG. 8A** fully enclosed within segmented sheath member **820.** The stent is simultaneously crimped and sheathed by segments **830** of sheath member **820** held within contoured depressions formed in crimping members of a radial compression device. Such a device is described above and illustrated in **FIGS. 1A-7B.** Full enclosure of the crimped stent may be accomplished using a crimping and sheathing system such as is seen in **FIGS. 1A-2B, 3A** and **3B, 6A** and **6B,** and **7A** and **7B.** In another embodiment, the sheath segments may partially enclose the crimped stent as would result when using a system such as that seen in **FIGS. 4A** and **4B,** and **5A** and **5B.**

As illustrated in **FIGS. 8A** and **8B,** sheath member **820** comprises six sheath segments **830;** however, the number of sheath segments is variable. For the present embodiment, sheath member **820** is preferably formed from one or more materials nonreactive with a coated stent, for example polypropylene, polyethylene, a nylon/polyethylene blend, PTFE (polytetrafluoroethylene), or a suitable polycarbonate. In another embodiment, the sheath member may comprise another appropriate biocompatible material, including, but not limited to, stainless steel and nitinol. Sheath segments **830** may be formed by cutting evenly spaced longitudinal slits into a section of tubing, or each segment may be formed individually by, for example, extruding, molding, cutting, or stamping the segments.

In the present embodiment, stent **810** is delivered to a treatment site using catheter **840,** which includes inner member **842** and outer member **844.** Stent **810** is crimped about inner member **842.** Outer member **844** is drawn over both stent **810** and sheath member **820** during manufacture of the system and serves as an outer sheath that holds stent **810** in a compressed configuration and maintains sheath segments **830** in place about the crimped stent.

As shown in **FIGS. 8A** and **8B,** sheath member **820** is longer than the stent it partially encloses but substantially shorter than inner member **842.** In the present embodiment, a proximal portion of sheath member **820** is attached to a distal portion of inner member **842** using, for example, an adhesive or heat bonding. Where sheath segments **830** are cut from a section of tubing, an uncut proximal portion of the tubing may be attached to the inner member. Where the sheath segments are formed individually, they may be individually attached to the inner member. In another embodiment, sheath member **820** may be approximately the same length as inner member **842,** with sheath segments **830** formed into the distal end of the sheath member. In this embodiment, the sheath member is not attached to the inner member and is held in position relative to the inner member by a handle attached to a proximal end of the system.

**FIG. 8A** shows the system ready for delivery into the vascular system of a patient. Outer member **844** is shown in cross section. Inner member **842** and sheath member **820** are shown in full except for a portion of sheath member **820** that is cut away to reveal crimped stent **810.** Sheath segments **830** protect crimped stent **810** from abrasion as outer member **844** is withdrawn to deliver stent **810** at the treatment site. The sheath segments also hold stent **810** in position about inner member **842** as outer member **844** is withdrawn, protecting the stent from being longitudinally compressed or otherwise distorted as the outer member passes over the stent. One skilled in the art will appreciate that stent **810** may be deployed by extending inner member **842** or by pushing on a proximal end of stent **810** in combination with or instead of withdrawing outer member **844.** A stent experiences less force during deployment using the present invention than using a conventional delivery system because the segmented sheath effectively reinforces the stent.

**FIG. 8B** shows the system partially deployed, with outer member **844** partially retracted. Once outer member **844** has been retracted, stent **810** expands, thereby expanding the portion of sheath member **820** enclosing the stent. As sheath member **820** expands, sheath segments **830** separate and are pressed against the wall of the target vessel by the expanded stent. Sheath member **820** is preferably segmented such that the sheath segments extend proximal to the proximal edge of crimped stent **810.** For example, where the stent is approximately 80 millimeters in length, the sheath member may be segmented to a point approximately 1 centimeter proximal to the proximal edge of the stent. This allows sheath segments **830** to fully separate along the entire length of the stent, thereby allowing crimped stent **810** to expand along the entire length of the stent once outer member **844** has been fully retracted.

Once stent **810** has fully expanded, catheter **840** may be removed from the patient, simultaneously withdrawing inner member **842** from within stent **810** and sheath member **820** from the outside of stent **810,** leaving the stent in place within the vessel. Because the separated sheath segments cover only a fraction of the outer surface of the expanded stent and exert only as much pressure on the stent as is produced by the expansive force of the stent against the inner wall of the vessel, little or no damage is caused to the stent or stent coating as the sheath segments are withdrawn over the outer surface of the expanded stent.

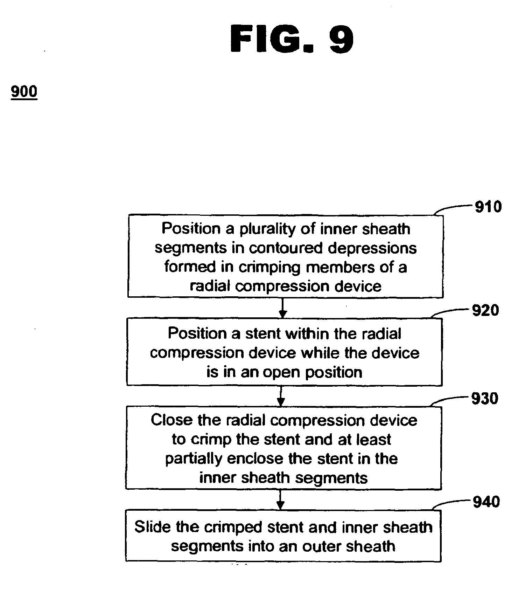

Yet another aspect of the present invention is a method of crimping and sheathing a stent. **FIG. 9** shows a flow diagram of one embodiment of the method in accordance with the present invention.

A plurality of inner sheath segments are positioned in contoured depressions formed in crimping members of a radial compression device **(Block 910)**. The radial compression device may be one such as has been described above and illustrated in **FIGS. 1A-7B,** with the crimping members forming a central aperture into which an expanded stent is inserted for crimping.

The sheath segments may be positioned in the contoured depressions either simultaneously or individually. In the present embodiment, prior to inserting the segments into the device, the segments are attached adjacent to a distal end of a catheter inner member. In this embodiment, the segments are slid into the contoured depressions as a group while the compression device is either open or closed, at the same time positioning a distal portion of the inner member within the central aperture formed by the crimping members. Where the device is closed during positioning of the segments, it must be opened prior to positioning a stent within the device.

Alternatively, the sheath segments may be positioned within the contoured depressions, either simultaneously or individually, while the device is open, and a catheter inner member may be separately positioned within the central aperture of the compression device. The segments may be attached to the inner member when the compression device is closed to crimp a stent. In still another alternative, the inner sheath segments may be formed into a distal portion of an elongated section of tubing, and an inner member may be threaded into the tubing either before or after the sheath segments are positioned in the contoured depressions of the crimping members.

A stent is positioned within the radial compression device while the device is in an open position **(Block 920).** In the present embodiment, an expanded stent is inserted into the device over the distal portion of the inner member that has already been positioned within the central aperture of the compression device. The stent is thereby positioned within the sheath segments, which when held by the crimping members form a roughly cylindrical structure. Thus, the sheath segments, the stent, and the inner member are all positioned coaxial to one another, with the sheath segments forming the outer layer, the catheter inner member forming the inner layer, and the stent sandwiched between. One skilled in the art will appreciate that the order of placing the sheath segments, the inner member, and the stent into the compression device may be varied; however, the order described above is believed to be the most efficient.

The radial compression device is closed to crimp the stent and to at least partially enclose the stent in the inner sheath segments (Block 930). As previously described, the contoured depressions and the sheath segments they hold are rotated into the central aperture of the compression device to reduce the diameter of the aperture, thereby crimping the stent. At the same time, the sheath segments are positioned around the crimped stent, thereby at least partially enclosing the stent. The degree of enclosure will depend upon the design of the individual crimping members and the percentage of crimping members in the compression device that hold sheath segments.

The crimped stent and inner sheath segments are slid into an outer sheath **(Block 940).** In the present embodiment, the sheath segments, crimped stent, and catheter inner member are simultaneously withdrawn from the closed compression device into a proximal portion of the catheter outer member. The outer member serves as an outer sheath to hold the stent in a compressed configuration and the sheath segments in place about the crimped stent.

presently considered to be preferred, various changes and modifications can be The scope of the invention is indicated in the appended claims,

## Claims

1. A system for crimping and sheathing a stent, comprising:
a radial compression device (110, 310, 410, 510, 610, 710) including a plurality of crimping members (120, 320, 420, 520, 620, 720), at least a plurality of the crimping members (120, 320, 420, 520, 620, 720) each including at least one contoured depression (122, 322, 422, 522, 622, 722); and
**characterized in that** the system further comprises
a plurality of inner sheath segments (130, 330, 430, 530, 630, 730) received in the contoured depressions (122, 322, 422, 522, 622, 722);
wherein the contoured depressions (122, 322, 422, 522, 622, 722) are adapted to hold the sheath segments (130, 330, 430, 530, 630, 730) while the compression device (110, 310, 410, 510, 610, 710) moves between an open position and a closed position.

2. The system of claim 1 wherein the crimping members (120, 320, 420, 520, 620, 720) are disposed about the circumference of a circle.

3. The system of claim 1 or 2 wherein each crimping member (120, 320, 420, 520, 620, 720) rotates about a different longitudinal axis as the radial compression device (110, 310, 410, 510, 610, 710) moves between an open and a closed position.

4. The system of any of claims 1 to 3 wherein as the radial compression device (110, 310, 410, 510, 610, 710) moves from an open position to a closed position, the contoured depression (122, 322, 422, 522, 622, 722) of each crimping member (120, 320, 420, 520, 620, 720) extends into a central aperture (124) formed by the crimping members.

5. The system of nay of claims 1 to 4 wherein the crimping members (120, 320, 420, 520, 620, 720) form a central aperture (124) sized to receive an expanded stent when the radial compression device (110, 310, 410, 510, 610, 710) is in an open position, and wherein the crimping members (120, 320, 420, 520, 620, 720) form a substantially cylindrical central aperture (124) sized to crimp the stent when the radial compression device (110, 310, 410, 510, 610, 710) is in a closed position.

6. The system of any of claims 1 to 5 wherein each contoured depression serves as a shape lock to hold a sheath segment.

7. The system of any of claims 1 to 6 wherein the sheath segments (130, 330, 430, 530, 630, 730) are held within the contoured depressions (122, 322, 422, 522, 622, 722) using a method selected from a group consisting of suction, adhesion, and electrostatic attraction.

8. The system of any of claims 1 to 7 wherein the sheath segments (130, 330, 430, 530, 630, 730) are positioned to at least partially enclose a crimped stent when the radial compression device (110, 310, 410, 510, 610, 710) is in a closed position.

9. The system of any of claims 1 to 8 wherein the contoured depressions (122, 322, 422, 522, 622, 722) allow the sheath segments (130, 330, 430, 530, 630, 730) to be slidably removed from the radial compression device (110, 310, 410, 510, 610, 710) when the device is in a closed position.

10. The system of any of claims 1 to 9 wherein each crimping member (120, 320, 420, 520, 720) is substantially trapezoidal in cross section.

11. The system of any of claims 1 to 9 wherein each crimping member (620) is substantially triangular in cross section.

12. A system for treating a vascular condition, comprising:
a crimped stent (810) **characterised in that** it is at least partially enclosed within a segmented sheath member (820), wherein the stent (810) was simultaneously crimped and sheathed with the system for crimping and sheathing a stent of claim 1.

13. The system of claim 12, further comprising:
a catheter (840) having an inner member (842) and an outer member (844).

14. The system of claim 13 wherein the stent (810) is crimped about the inner member (842).

15. The system of claim 13 or 14 wherein the sheath member (820) is attached to the inner member (842).

16. The system of any of claims 13 to 15 wherein the outer member (844) serves as an outer sheath.

17. A method of crimping and sheathing a stent using the system for crimping and sheathing a stent of claim 1, the method comprising:
positioning a plurality of inner sheath segments in contoured depressions formed in crimping members of a radial compression device;
positioning a stent within the radial compression device while the device is in an open position;
closing the radial compression device to crimp the stent and to at least partially enclose the stent in the inner sheath segments; and
sliding the crimped stent and inner sheath segments into an outer sheath.

18. The method of claim 17, the method further comprising:
closing the radial compression device prior to positioning the inner sheath segments in the contoured depressions formed in the crimping members; and
opening the radial compression device prior to positioning the stent within the radial compression device.

19. The method of claim 17 or 18 wherein the plurality of inner sheath segments are attached adjacent to a distal end of an inner member, and wherein positioning a plurality of inner sheath segments in contoured depressions formed in crimping members of a radial compression device further comprises positioning a distal portion of the inner member within a central aperture of the radial compression device.

20. The method of claim 19 wherein positioning a stent within the open radial compression device further comprises positioning the stent over the distal portion of the inner member.

## Patentansprüche

1. System zum Quetschen und Einhüllen eines Stents, welches umfasst:
eine radiale Komprimierungsvorrichtung (110, 310, 410, 510, 610, 710), einschließlich mehrerer Quetschelemente (120, 320, 420, 520, 620, 720), wobei mindestens mehrere der Quetschelemente (120, 320, 420, 520, 620, 720) jeweils mindestens eine Profilmulde (122, 322, 422, 522, 622, 722) aufweisen; **dadurch gekennzeichnet, dass** das System des Weiteren mehrere innere Hüllsegmente (130, 330, 430, 530, 630, 730) aufweist, die in den Profilmulden (122, 322, 422, 522, 622, 722) aufgenommen sind;
wobei die Profilmulden (122, 322, 422, 522, 622, 722) angepasst sind, um die Hüllsegmente (130, 330, 430, 530, 630, 730) zu halten, während sich die Komprimierungsvorrichtung (110, 310, 410, 510, 610, 710) zwischen einer offenen Position und einer geschlossenen Position bewegt.

2. System nach Anspruch 1, wobei die Quetschelemente (120, 320, 420, 520, 620, 720} um den Umfang eines Kreises angeordnet sind.

3. System nach Anspruch 1 oder 2, wobei ein jeweiliges Quetschelement (120,320, 420, 520, 620, 720) um eine andere Längsachse rotiert, wenn sich die radiale Komprimierungsvorrichtung (110, 310, 410, 510, 610, 710) zwischen einer offenen Position und einer geschlossenen Position bewegt.

4. System nach einem der Ansprüche 1 bis 3, wobei, wenn sich die radiale Komprimierungsvorrichtung (110, 310, 410, 510, 610, 710) von einer offenen Position in eine geschlossene Position bewegt, die Profilmulde (122, 322, 422, 522, 622, 722) eines jeweiligen Quetschelementes (120, 320, 420, 520, 620, 720) in eine zentrale Öffnung (124) verläuft, die von den Quetschelementen ausgebildet wird.

5. System nach einem der Ansprüche 1 bis 4, wobei die Quetschelemente (120, 320, 420, 520, 620, 720) eine zentrale Öffnung (124) ausbilden, deren Größe angelegt ist, um einen expandierten Stent aufzunehmen, wenn die radiale Komprimierungsvorrichtung (110, 310, 410, 510, 610, 710) sich in einer offenen Position befindet, und wobei die Quetschelemente (120, 320, 420, 520, 620, 720) eine im Wesentlichen zylindrische zentrale Öffnung (124) ausbilden, deren Größe angelegt ist, um den Stent zu quetschen, wenn sich die radiale Komprimierungsvorrichtung (110, 310, 410, 510, 610, 710) in einer geschlossenen Position befindet.

6. System nach einem der Ansprüche 1 bis 5, wobei eine jeweilige Profilmulde als eine Formsperre dient, um ein Hüllensegment zu halten.

7. System nach einem der Ansprüche 1 bis 6, wobei die Hüllsegmente (130, 330, 430, 530, 630, 730) innerhalb der Profilmulden (122, 322, 422, 522, 622, 722) mithilfe eines Verfahrens gehalten werden, das aus einer Gruppe ausgewählt ist, welche aus Saugkraft, Adhäsion und elektrostatischer Anziehung besteht.

8. System nach einem der Ansprüche 1 bis 7, wobei die Hüllsegmente (130, 330, 430, 530, 630, 730) positioniert sind, um einen gequetschten Stent mindestens teilweise einzuschließen, wenn die sich radiale Komprimierungsvorrichtung (110, 310, 410, 510, 610, 710) in einer geschlossenen Position befindet.

9. System nach einem der Ansprüche 1 bis 8, wobei die Profilmulden (122, 322, 422, 522, 622, 722) den Hüllensegmenten (130, 330, 430, 530, 630, 730) erlauben, verschiebbar von der radialen Komprimierungsvorrichtung (110, 310, 410, 510, 610, 710) entfernt zu werden, wenn sich die Vorrichtung in einer geschlossenen Position befindet.

10. System nach einem der Ansprüche 1 bis 9, wobei ein jeweiliges Quetschelement (120, 320, 420, 520, 720) im Querschnitt im Wesentlichen trapezförmig ist.

11. System nach einem der Ansprüche 1 bis 9, wobei ein jeweiliges Quetschelement (620) im Querschnitt im Wesentlichen dreieckig ist.

12. System zum Behandeln vaskulärer Beschwerden, umfassend:
einen gequetschten Stent (810), der **dadurch gekennzeichnet ist, dass** er mindestens teilweise innerhalb eines segmentierten Hüllelementes (820) eingeschlossen ist, wobei der Stent (810) mit dem System zum Quetschen und Einhüllen eines Stents nach Anspruch 1 gleichzeitig gequetscht und eingehüllt worden ist.

13. System nach Anspruch 12, des Weiteren umfassend:
einen Katheter (840) mit einem inneren Element (842) und einem äußeren Element (844).

14. System nach Anspruch 13, wobei der Stent (810) um das innere Element (842) gequetscht wird.

15. System nach Anspruch 13 oder 14, wobei das Hüllelement (820) an dem inneren Element (842) befestigt ist.

16. System nach einem der Ansprüche 13 bis 15, wobei das äußere Element (844) als äußere Hülle dient.

17. Verfahren zum Quetschen und Einhüllen eines Stents unter Verwendung des Systems zum Quetschen und Einhüllen eines Stents nach Anspruch 1, wobei das Verfahren umfasst:
Positionieren mehrerer innerer Hüllsegmente in Profilmulden, die in Quetschelementen einer radialen Komprimierungsvorrichtung ausgebildet sind;
Positionieren eines Stents in einer radialen Komprimierungsvorrichtung, während sich die Vorrichtung in einer offenen Position befindet;
Schließen der radialen Komprimierungsvorrichtung, um den Stent zu quetschen und den Stent mindestens teilweise in den inneren Hüllsegmenten einzuschließen; und
Verschieben des gequetschten Stents und der inneren Hüllsegmente in eine äußere Hülle.

18. Verfahren nach Anspruch 17, wobei das Verfahren des Weiteren umfasst:
Schließen der radialen Komprimierungsvorrichtung vor dem Positionieren der inneren Hüllsegmente in den in den Quetschelementen ausgebildeten Profilmulden; und
Öffnen der radialen Komprimierungsvorrichtung vor dem Positionieren des Stents in der radialen Komprimierungsvorrichtung.

19. Verfahren nach Anspruch 17 oder 18, wobei die mehreren inneren Hüllsegmente neben einem distalen Ende eines inneren Elementes angebracht sind und wobei das Positionieren mehrerer innerer Hüllsegmente in Quetschelementen einer radialen Komprimierungsvorrichtung ausgebildeten Profilmulden des Weiteren das Positionieren eines distalen Bereichs des inneren Elementes in einer zentralen Öffnung der radialen Komprimierungsvorrichtung umfasst.

20. Verfahren nach Anspruch 19, wobei das Positionieren eines Stents in der offenen radialen Komprimierungsvorrichtung des Weiteren das Positionieren des Stents über dem distalen Bereich des inneren Elementes umfasst.

## Revendications

1. Système pour sertir et gainer un stent, comprenant :
un dispositif de compression radiale (110,310,410, 510,610,710), incluant une série d'éléments de sertissage (120,320,420,520,620,720), une série au moins des éléments de sertissage (120,320,420,520,620,720)incluant chacun au moins une dépression profilée (122,322,422,522,622,722),
**caractérisé en ce que** le système comprend en outre :
une série de segments de gaine interne (130,330,430,530,630,730) reçus dans les dépressions profilées (122,322,422,522,622,722) ;
les dépressions profilées (122,322,422,522,622,722) étant adaptées à tenir les segments de gaine (130, 330, 430, 530, 630, 730) tandis que le dispositif de compression (110,310,410,510,610,710) se déplace entre une position ouverte et une position fermée.

2. Système selon la revendication 1, dans lequel les éléments de sertissage (120, 320, 420, 520, 620, 720) sont disposés autour de la circonférence d'un cercle.

3. Système selon la revendication 1 ou 2, dans lequel chaque élément de sertissage (120,320,420,520,620,720) tourne autour d'un axe longitudinal différent tandis que le dispositif de compression radiale (110,310,410,510,610,710) se déplace entre une position ouverte et une position fermée.

4. Système selon l'une quelconque des revendications 1 à 3, dans lequel, tandis que le dispositif de compression radiale (110,310,410,510,610,710) se déplace entre une position ouverte et une position fermée, la dépression profilées (122,322,422,522,622,722) de chaque élément de sertissage (120,320,420,520,620,720) s'étend dans une ouverture centrale (124) formées par les éléments de sertissage.

5. Système selon l'une quelconque des revendications 1 à 4, dans lequel les éléments de sertissage (120,320,420,520,620,720) forment une ouverture centrale (124) dimensionnée pour recevoir un stent dilaté tandis que le dispositif de compression radiale (110,310,410, 510,610,710) est en position ouverte et dans lequel les éléments de sertissage (120,320,420,520,620,720) forment une ouverture centrale (124) sensiblement cylindrique dimensionnée pour sertir le stent lorsque le dispositif de compression radiale (110,310,410,510,610,710) est en position fermée.

6. Système selon l'une quelconque des revendications 1 à 5, dans lequel chaque dépression profilée sert de forme de blocage pour tenir un segment de gaine.

7. Système selon l'une quelconque des revendications 1 à 6, dans lequel les segments de gaine (130,330,430,530,630,730) sont tenus au sens des dépressions profilées (122,322,422,522,622,722) en utilisant une technique sélectionnée dans un groupe consistant en l'aspiration, l'adhérence ou l'attraction électrostatique.

8. Système selon l'une quelconque des revendications 1 à 7, dans lequel les segments de gaine (130,330,430,530,630,730) sont positionnés de manière à enclore au moins partiellement un stent serti lorsque le dispositif de compression radiale (110,310,410, 510,610,710) est en position fermée.

9. Système selon l'une quelconque des revendications 1 à 8, dans lequel les dépressions profilées (122, 322, 422, 522, 622, 722) permettent aux segments de gaine (130, 330, 430, 530, 630, 730) d'être enlevés par coulissement du dispositif de compression radiale (110,310,410, 510,610,710) lorsque le dispositif est en position fermée.

10. Système selon l'une quelconque des revendications 1 à 9, dans lequel chaque élément de sertissage (120,320,420,520,720) est de section transversale sensiblement trapézoïdale.

11. Système selon l'une quelconque des revendications 1 à 9, dans lequel chaque élément de sertissage (620) est de section transversale sensiblement triangulaire.

12. Système pour traiter un état vasculaire, comprenant :
un stent serti **caractérisé en ce qu'**il est au moins partiellement enclos au sein d'un élément de gaine segmenté (820), le stent (810) ayant été simultanément serti et gainé au moyen du système de sertissage et gainage de stent selon la revendication 1.

13. Système selon la revendication 12, comprenant en outre :
un cathéter (840) ayant un élément interne (842) et un élément externe (844).

14. Système selon la revendication 13, dans lequel le stent (810) est serti autour de l'élément interne (842).

15. Système selon la revendication 13 ou 14, dans lequel l'élément de gaine (820) est fixé à l'élément interne (842).

16. Système selon l'une quelconque des revendications 13 à 15, dans lequel l'élément externe (844) sert de gaine externe.

17. Procédé de sertissage et de gainage d'un stent utilisant le système de sertissage et de gainage de stent selon la revendication 1, ledit procédé comprenant :
le positionnement d'une série de segments de gaine interne dans des dépressions profilées formées dans des éléments de sertissage d'un dispositif de compression radiale ;
le positionnement d'un stent au sein du dispositif de compression radiale tandis que le dispositif est en position ouverte ;
la fermeture du dispositif de compression radiale pour sertir le stent et pour au moins enclore partiellement le stent dans les segments de gaine interne ; et
le fait de faire coulisser le stent serti et les segments de gaine interne dans une gaine externe.

18. Procédé selon la revendication 17, ledit procédé comprenant en outre :
la fermeture du dispositif de compression radiale avant le positionnement des segments de gaine interne dans les dépressions profilées formées dans les éléments de sertissage ; et
l'ouverture du dispositif de compression radiale avant le positionnement du stent au sein du dispositif de compression radiale.

19. Procédé selon la revendication 17 ou 18, dans lequel la série de segments de gaine interne sont fixés au voisinage d'une extrémité distale d'un élément interne et dans lequel le positionnement d'une série de segments de gaine interne dans des dépressions profilées formées dans des éléments de sertissage d'un dispositif de compression radiale comprend, en outre, le positionnement d'une portion distale de l'élément interne au sein d'une ouverture centrale du dispositif de compression radiale.

20. Procédé selon la revendication 19, dans lequel le positionnement d'un stent au sein du dispositif de compression radiale ouvert comprend, en outre, le positionnement du stent par-dessus la portion distale de l'élément interne.
